# EUROPEAN PATENT APPLICATION

(11) **EP 4 317 159 A1**
(43) Date of publication of application: **07.02.2024**
(21) Application number: 22779116.7
(22) Date of filing: 01.04.2022
(51) Int. Cl.: C07D 471/04

(54) **METHOD FOR PREPARING PHOSPHODIESTERASE INHIBITOR**

(30) Priority: 01.04.2021 CN 202110356848
(71) Applicant: Transthera Sciences (Nanjing), Inc., Jiangbei New Area Nanjing Jiangsu 210032 (CN)
(72) Inventor: LI, Lin, Nanjing, Jiangsu 210032 (CN); WANG, Hao, Nanjing, Jiangsu 210032 (CN)
(74) Representative: Grünecker Patent- und Rechtsanwälte PartG mbB
(86) International application number: PCT/CN2022/084704
(87) International publication number: WO 2022/206936

(57) **Abstract**

The present invention belongs to the technical field of medicine, and specifically relates to the preparation process of compounds, as represented by formulas (I) and (I'), and intermediates thereof. The preparation process of the present invention has lower production cost and generates less three wastes.

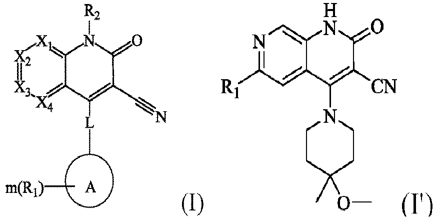

## Description

### TECHNICAL FIELD

The present invention belongs to the technical field of pharmaceutics, and particularly relates to a preparation method and a preparation intermediate for a phosphodiesterase inhibitor.

### BACKGROUND

Phosphodiesterase 9 is an important member of the PDE family, with very high selectivity for cGMP, and its inhibitors are used in the treatment of diseases associated with cognitive impairments caused by central nervous system disorders, such as senile dementia, schizophrenia, and diseases of the neurodegenerative process in the brain.

6-Ethyl-4-(4-methoxy-4-methylpiperidin-1 -yl)-2-oxo-1,2-dihydro-1,7-naphthyridine-3-carbonitrile (i.e., compound 107 in Example 71 of WO2019062733A1) is an inhibitor of PDE9.

### Example 71 of WO2019062733A1 discloses a preparation method comprising the following steps:

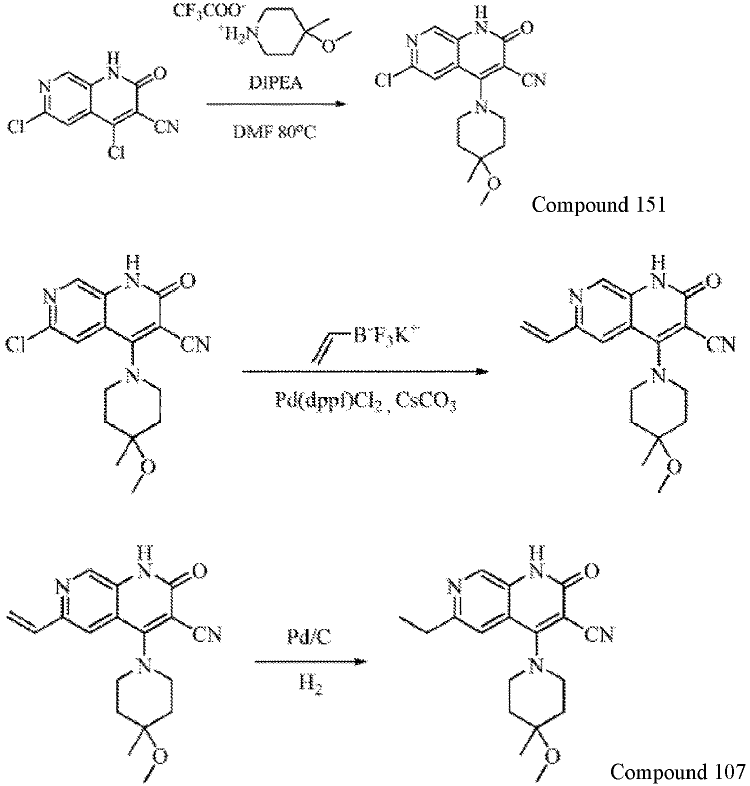

In introducing substituents to locants 4 and 6 of the 1,7-naphthyridine ring, a coupling reaction and a nucleophilic substitution reaction are needed, resulting in a long reaction process and a low total yield. In the preparation of compound 107, the intermediate 4,6-dichloro-2-oxo-1,2-dihydro-1,7-naphthyridine-3-carbonitrile (whose structure is shown below) is needed as the starting material of the synthesis.

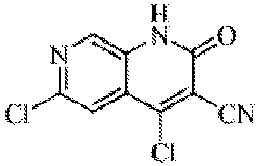

### Preparation Example 2 of WO2019062733A1 discloses a preparation method for the intermediate 4,6-dichloro-2-oxo-1,2-dihydro-1,7-naphthyridine-3-carbonitrile,

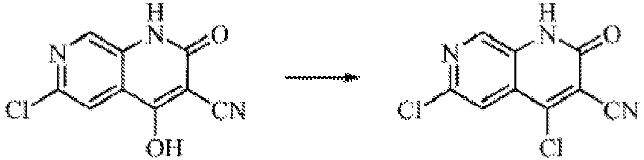

in which 15 mL of phosphorus oxychloride (having a density of 1.64 g/cm³ and a molecular weight of 153.33 g/mol; hence 24.6 g, 0.16 mol, 7 equivalents) is used, and about 0.7 equivalents of the desired product is obtained. That is to say, to obtain 1 equivalent of 6-ethyl-4-(4-methoxy-4-methylpiperidin-1-yl)-2-oxo-1,2-dihydro-1,7-naphthyridine-3-carbonitrile, the method disclosed in WO2019062733A1 needs to use about 30 equivalents of phosphorus oxychloride.

WO2020182076A1 discloses an identical method for preparing the intermediate 4,6-dichloro-2-oxo-1,2-dihydro-1,7-naphthyridine-3-carbonitrile (page 28, Preparation Example 1, step 3).

WO2020182076A1 also discloses a preparation method for 6-ethyl-4-chloro-2-oxo-1,2-dihydro-1,7-naphthyridine-3-carbonitrile (page 29, Preparation Example 2, step 3). According to this method, to prepare 1 equivalent of 6-ethyl-4-chloro-2-oxo-1,2-dihydro-1,7-naphthyridine-3-carbonitrile, 5.13 equivalents of phosphorus oxychloride are needed, and a chromatography separation method in addition.

Phosphorus oxychloride is highly toxic and explosive and easily causes environmental pollution. It is therefore desirable to minimize the use of phosphorus oxychloride in the synthesis process. In actual pharmaceutical production, a short process is also desirable, and purification steps are avoided whenever possible so as to improve the yield. Therefore, in the subsequent research of scale-up synthesis, the synthesis process still needs improvement that can largely reduce production costs and the generation of three types of industrial waste, improving the safety of the process.

### SUMMARY

The present invention aims to provide a preparation method for a compound of formula (I) and provide a preparation intermediate, and to further provide a preparation method for a compound of formula (I') and provide a preparation intermediate.

The present invention provides a preparation method for a compound of formula (I), comprising the following steps: 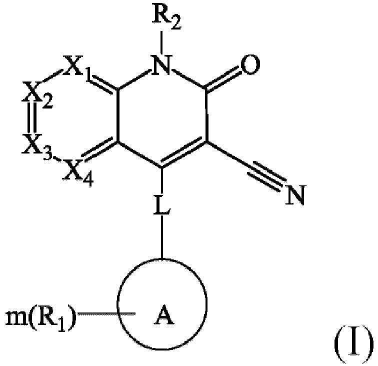
step (C'): in an organic solvent, carrying out a halogenation reaction of (I-4) with a halogenation reagent to give (I-5): 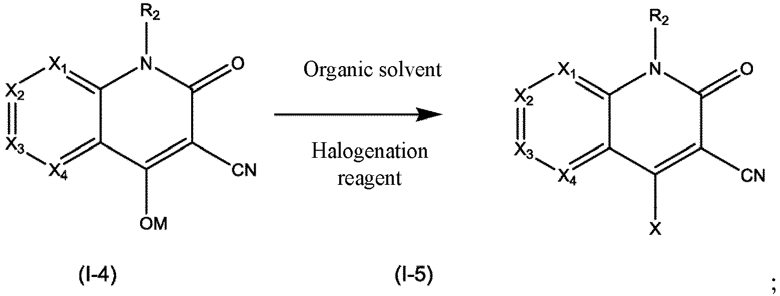
step (D'): in an organic solvent, carrying out a nucleophilic substitution reaction of (I-5) with (I-6) and a base to give (I'): 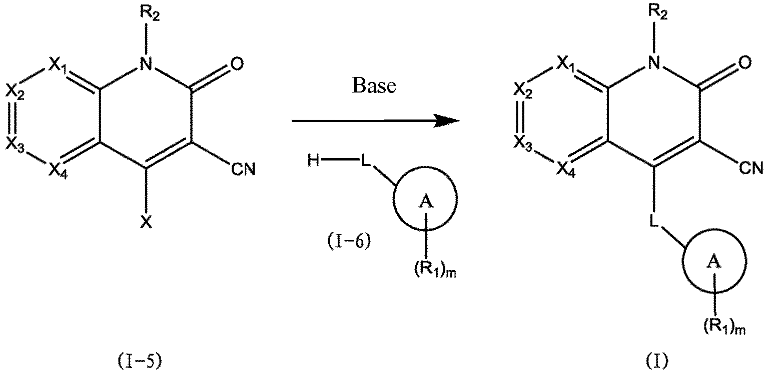
wherein X is a halogen atom; M is an alkali metal ion;
X₁ is CH, X₂ is N, X₃ is CR₃, and X₄ is CH;
R₃ is selected from hydrogen, amino, cyano, halogen, carboxyl, C₁₋₄ alkyl, C₁₋₄ alkoxy, C₁₋₄ alkylamino, (C₁₋₄ alkyl)₂amino, morpholinyl, C₂₋₆ alkenyl, C₁₋₄ alkylcarbonyl, C₁₋₄ alkylaminocarbonyl, (C₁₋₆ alkyl)₂aminocarbonyl, C₁₋₄ alkylsulfonyl, C₁₋₄ alkylthio, aminocarbonyl, cyclopropyl, azetidinyl and piperazinyl,
   a) wherein the C₁₋₄ alkyl, C₁₋₄ alkoxy, C₁₋₄ alkylamino, (C₁₋₄ alkyl)₂amino, C₂₋₆ alkenyl, C₁₋₄ alkylcarbonyl, C₁₋₄ alkylaminocarbonyl, (C₁₋₆ alkyl)₂aminocarbonyl, C₁₋₄ alkylsulfonyl, C₁₋₄ alkylthio and aminocarbonyl are unsubstituted or optionally substituted with one or more (e.g., 1, 2, 3, 4 or 5, provided that a valence balance is achieved) groups independently selected from hydroxyl, amino, halogen, C₁₋₄ alkyl, C₁₋₄ alkoxy, C₁₋₄ alkylamino, (C₁₋₄ alkyl)₂amino, cyclopropyl, C₁₋₄ alkylcarbonyloxy, and 4-6 membered heterocyclyl which is unsubstituted or substituted with C₁₋₄ alkyl; and
   b) wherein the cyclopropyl, azetidinyl, morpholinyl and piperazinyl are unsubstituted or optionally substituted with one or more (e.g., 1, 2, 3, 4 or 5, provided that a valence balance is achieved) groups independently selected from hydroxyl, halogen, C₁₋₄ alkyl, C₁₋₄ alkoxy, cyclopropyl, amino, C₁₋₄ alkylamino, (C₁₋₄ alkyl)₂amino, and C₁₋₄ alkylcarbonyloxy;
preferably, R₃ is selected from hydrogen, halogen, C₁₋₄ alkyl, C₁₋₄ alkoxy, morpholinyl, C₂₋₆ alkenyl, C₁₋₄ alkylaminocarbonyl, (C₁₋₄ alkyl)₂aminocarbonyl, and aminocarbonyl,
   a) wherein the C₁₋₄ alkyl, C₁₋₄ alkoxy, C₂₋₆ alkenyl, C₁₋₄ alkylaminocarbonyl, (C₁₋₄ alkyl)₂aminocarbonyl, and aminocarbonyl are unsubstituted or optionally substituted with one or more (e.g., 1, 2, 3, 4 or 5, provided that a valence balance is achieved) groups independently selected from hydroxyl, C₁₋₄ alkoxy, cyclopropyl, amino, C₁₋₄ alkylamino, (C₁₋₄ alkyl)₂amino, and 4-6 membered heterocyclyl which is unsubstituted or substituted with C₁₋₄ alkyl; and
   b) wherein the morpholinyl is unsubstituted or optionally substituted with one or more (e.g. 1, 2, 3, 4 or 5, provided that a valence balance is achieved) groups independently selected from hydroxyl, C₁₋₄ alkoxy, cyclopropyl, amino, C₁₋₄ alkylamino, and (C₁₋₄ alkyl)₂amino;
more preferably, R₃ is selected from hydrogen, halogen, C₁₋₄ alkyl, C₁₋₄ alkoxy, C₂₋₆ alkenyl, C₁₋₄ alkylaminocarbonyl, and aminocarbonyl,
wherein the C₁₋₄ alkyl, C₁₋₄ alkoxy, C₂₋₆ alkenyl, C₁₋₄ alkylaminocarbonyl, and aminocarbonyl are unsubstituted or optionally substituted with one or more (e.g., 1, 2, 3, 4 or 5, provided that a valence balance is achieved) groups independently selected from hydroxyl, C₁₋₄ alkoxy, cyclopropyl, amino, C₁₋₄ alkylamino, (C₁₋₄ alkyl)₂amino, and 4-6 membered heterocyclyl which is unsubstituted or substituted with C₁₋₄ alkyl;
L is a bond;
ring A is 4-7 membered monocyclic heterocyclyl or 7-12 membered spiro-heterocyclyl, wherein a heteroatom of the 4-7 membered monocyclic heterocyclyl is selected from N; a heteroatom of the 7-12 membered spiro-heterocyclyl is selected from one of or a combination of two of O and N; the 7-12 membered spiro-heterocyclyl contains at least one N, and ring A is linked to L by the N atom;
preferably, ring A is a 4-7 membered monocyclic heterocyclyl, wherein a heteroatom of the 4-7 membered monocyclic heterocyclyl is selected from N, and ring A is linked to L by the N atom;
more preferably, ring A is selected from 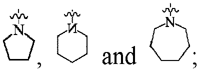
particularly more preferably, ring A is 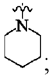
each R₁ is independently selected from hydrogen, hydroxyl, cyano, halogen, C₁₋₄ alkyl, C₁₋₄ alkoxy, pyrazolyl, thiazolyl and triazolyl, wherein the C₁₋₄ alkyl, C₁₋₄ alkoxy, pyrazolyl, thiazolyl and triazolyl are unsubstituted or substituted with hydroxyl;
preferably, each R₁ is independently selected from hydrogen, C₁₋₄ alkyl, C₁₋₄ alkoxy, pyrazolyl, thiazolyl and triazolyl, wherein the C₁₋₄ alkyl, C₁₋₄ alkoxy, pyrazolyl, thiazolyl and triazolyl are unsubstituted or substituted with hydroxyl;
m is 0, 1 or 2;
R₂ is selected from hydrogen.

The present invention provides a preparation method for a compound of formula (I'), comprising the following steps: 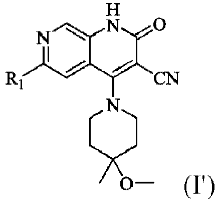
step (C): in an organic solvent, carrying out a halogenation reaction of (I'-4) with a halogenation reagent to give (I'-5): 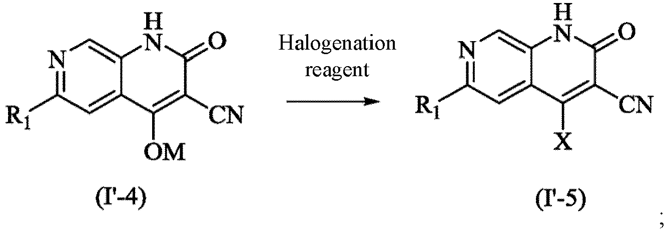
step (D): in an organic solvent, carrying out a nucleophilic substitution reaction of (I'-5) with (I'-6) and a base to give (I'): 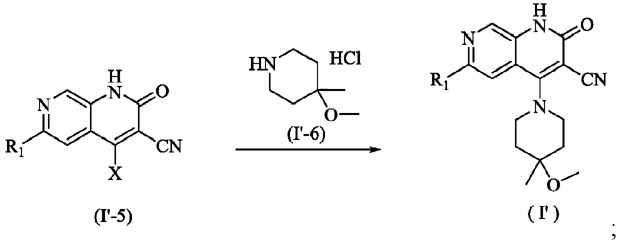
wherein R₁ is C₁₋₆ alkyl;
X is a halogen atom;
M is an alkali metal ion.

In one embodiment of the present invention, provided is a preparation method for a compound of formula (I'), comprising the following steps:
step (C): in an organic solvent, carrying out a halogenation reaction of (I'-4) with a halogenation reagent to give (I'-5): 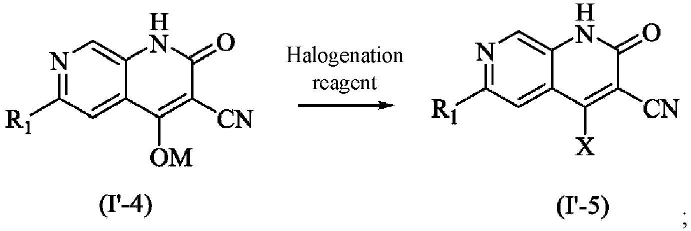
step (D): in an organic solvent, carrying out a nucleophilic substitution reaction of (I'-5) with (I'-6) and a base to give (I'): 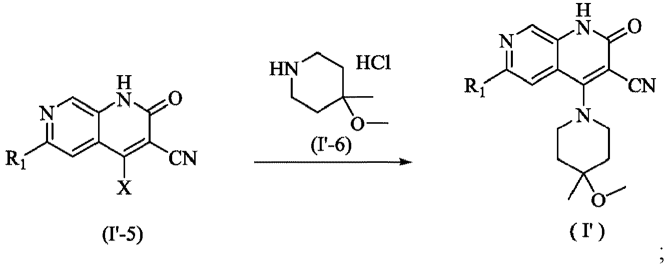
wherein R₁ is C₁₋₆ alkyl;
X is a halogen atom;
M is K⁺, Na⁺, Li⁺ or Cs⁺.

In one embodiment of the present invention, the preparation method for the compound of formula (I') further comprises step (B): in an organic solvent, carrying out a condensation reaction of (I'-3) with a base to give (I'-4): 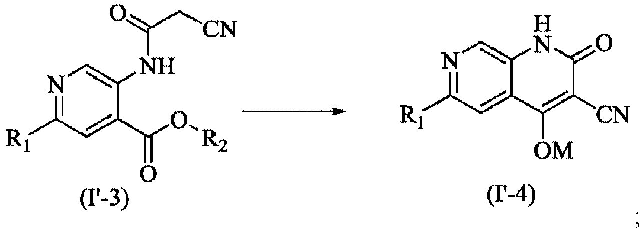
wherein R₁ is C₁₋₆ alkyl, preferably methyl or ethyl;
R₂ is C₁₋₆ alkyl, preferably methyl or ethyl;
M is K⁺, Na⁺, Li⁺ or Cs⁺, preferably K⁺ or Na⁺.

In one embodiment of the present invention, the preparation method for the compound of formula (I') further comprises step (A): in an organic solvent, carrying out a condensation reaction of (I'-1) and (I'-2) with a coupling reagent to give (I'-3): 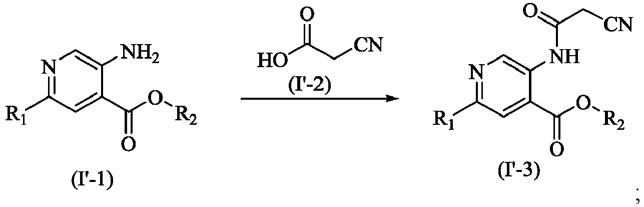
wherein R₁ is C₁₋₆ alkyl, preferably methyl or ethyl;
R₂ is C₁₋₆ alkyl, preferably methyl or ethyl.

In one embodiment of the present invention,
the coupling reagent in step (A) is one or more of 1-(3-dimethylaminopropyl)-3-ethylcarbodiimide hydrochloride, 2-(7-oxidobenzotriazole)-N,N,N',N'-tetramethyluronium hexafluorophosphate, benzotriazole-1-yloxytris(dimethylamino)phosphonium hexafluorophosphate and dicyclohexylcarbodiimide;
the base in step (B) is one or more of potassium hydroxide, sodium hydroxide, sodium methoxide, sodium ethoxide, sodium tert-butoxide, potassium tert-butoxide and potassium methoxide;
the halogenation reagent in step (C') and step (C) is one or more of phosphorus oxychloride, thionyl chloride, sulfuryl chloride, phosphorus trichloride, phosphorus pentachloride, phosphorus tribromide, phosphorus oxybromide, triphosgene and oxalyl chloride;
the base in step (D') and step (D) is one or more of triethylamine, N,N-diisopropylethylamine, sodium methoxide, sodium ethoxide, sodium tert-butoxide and potassium tert-butoxide.

In one embodiment of the present invention,
the base in step (D') and step (D) is triethylamine.

In one embodiment of the present invention,
the organic solvent in step (A) is one or more of dichloromethane, N,N-dimethylformamide, N,N-dimethylacetamide and tetrahydrofuran, preferably dichloromethane;
the organic solvent in step (B) is one or more of ethanol, tetrahydrofuran, 2-methyltetrahydrofuran, N,N-dimethylformamide and N,N-dimethylacetamide, preferably tetrahydrofuran;
the organic solvent in step (C') and step (C) is one or more of acetonitrile, tetrahydrofuran, 2-methyltetrahydrofuran, chloroform and 1,2-dichloroethane, preferably acetonitrile;
the organic solvent in step (D') and step (D) is one or more of ethanol, tetrahydrofuran, 2-methyltetrahydrofuran, N,N-dimethylformamide and N,N-dimethylacetamide, preferably ethanol.

In one embodiment of the present invention,
the coupling reagent in step (A) is 1-(3-dimethylaminopropyl)-3-ethylcarbodiimide hydrochloride;
the base in step (B) is one or more of potassium methoxide and sodium ethoxide;
the halogenation reagent in step (C') and step (C) is phosphorus oxychloride;
the base in step (D') and step (D) is triethylamine or N,N-diisopropylethylamine.

In one embodiment of the present invention,
in step (A), the organic solvent is used in an amount that is 2-20 times, preferably 5-12 times, the volume of formula (I'-1);
in step (B), the organic solvent is used in an amount that is 2-20 times, preferably 3-12 times, the volume of formula (I'-3);
in step (C'), the organic solvent is used in an amount that is 2-20 times, preferably 4-10 times, the volume of formula (I-4);
in step (C), the organic solvent is used in an amount that is 2-20 times, preferably 4-10 times, the volume of formula (I'-4);
in step (D'), the organic solvent is used in an amount that is 2-20 times, preferably 10-20 times, the volume of formula (I-5);
in step (D), the organic solvent is used in an amount that is 2-20 times, preferably 10-20 times, the volume of formula (I'-5).

In one embodiment of the present invention,
a molar ratio of (I'-1) to (I'-2) to the coupling reagent in step (A) is 1 :(0.5-2):(1-2);
a molar ratio of (I'-3) to the base in step (B) is 1:(1-5);
a molar ratio of (I-4) to the halogenation reagent in step (C') is 1:(1-5);
a molar ratio of (I'-4) to the halogenation reagent in step (C) is 1 :(1-5);
a molar ratio of (I-5) to (I-6) to the base in step (D') is 1:(0.5-2):(1-3);
a molar ratio of (I'-5) to (I'-6) to the base in step (D) is 1 :(0.5-2):(1-3).
In one embodiment of the present invention,
a molar ratio of (I-4) to the halogenation reagent in step (C') is 1 :(1-3);
a molar ratio of (I'-4) to the halogenation reagent in step (C) is 1 :(1-3).
In one embodiment of the present invention,
a molar ratio of (I-4) to the halogenation reagent in step (C') is 1:1, 1:1.1, 1:1.2, 1:1.5 or 1:2.3;
a molar ratio of (I'-4) to the halogenation reagent in step (C) is 1:1, 1:1.1, 1:1.2, 1:1.5 or 1:2.3.

The present invention also provides a preparation intermediate for the compound of formula (I'), having the following structural formula: 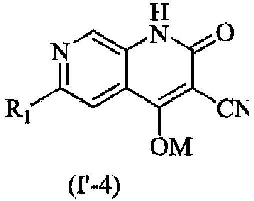
wherein R₁ is C₁₋₆ alkyl, preferably methyl or ethyl;
M is K⁺, Na⁺, Li⁺ or Cs⁺.

In one embodiment of the present invention, the preparation intermediate has the following structure:

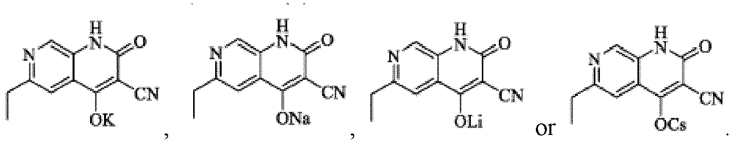

The present invention also provides a preparation intermediate for the compound of formula (I'), and the intermediate can be used in the synthesis of a medicament for treating or preventing PDE9-mediated related diseases.

### DETAILED DESCRIPTION

The "C₁₋₆ alkyl" described in the present invention refers to a linear or branched alkyl group derived by removing one hydrogen atom from a hydrocarbon moiety containing 1-6 carbon atoms, such as methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1-methyl-2-methylpropyl. The "C₁₋₄ alkyl" refers to the aforementioned examples that contain 1-4 carbon atoms.

The "C₁₋₄ alkoxy" described in the present invention refers to a group in which the "C₁₋₄ alkyl" defined above is linked to a parent molecule by an oxygen atom, i.e., a "C₁₋₄ alkyl-O-" group, such as methoxy, ethoxy, n-propoxy, isopropoxy, n-butoxy, and tert-butoxy.

The "C₁₋₄ alkylamino", "(C₁₋₄ alkyl)₂amino", "(C₁₋₄ alkyl)₂aminocarbonyl", "C₁₋₄ alkylsulfonyl" and "C₁₋₄ alkylthio" described in the present invention refer to C₁₋₄ alkyl-NH-, (C₁₋₄ alkyl)(C₁₋₄ alkyl)N-, (C₁₋₄ alkyl)(C₁₋₄ alkyl)N-C(O)-, C₁₋₄ alkyl-S(O)₂- and C₁₋₄ alkyl-S-, respectively.

The "4-6 membered heterocyclyl" described in the present invention refers to a 4-6 membered non-aromatic cyclic group in which at least one ring carbon atom is replaced by a heteroatom selected from O, S and N, preferably 1-3 heteroatoms. It also includes the cases where carbon atom(s), nitrogen atom(s), and sulfur atom(s) are substituted with oxygen.

The "halogen" described in the present invention refers to fluorine, chlorine, bromine, iodine, etc.

The "base" described in the present invention includes organic bases and inorganic bases. The organic bases include, but are not limited to, sodium tert-butoxide, potassium tert-butoxide, LiHMDS, sodium methoxide, sodium ethoxide, and potassium methoxide. The inorganic bases include, but are not limited to, potassium hydroxide, sodium hydroxide, magnesium hydroxide, and rubidium hydroxide.

The "alkali metal" described in the present invention refers to six metal elements in group IA of the periodic table, excluding hydrogen (H), i.e., lithium (Li), sodium (Na), potassium (K), rubidium (Rb), cesium (Cs) and francium (Fr).

The "times the volume" described in the present invention refers to the volume (mL) of solvent required to dissolve 1 g of substance; for example, if 10 mL of solvent is required to dissolve 1 g of the compound of formula (I'-1), it is referred to as 10 times the volume.

In particular, the present invention provides the following technical solutions:

Technical solution 1. A preparation method for a compound of formula (I), comprising the following steps: 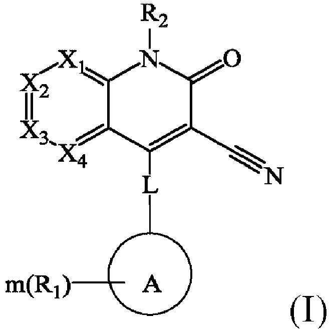
step (C'): in an organic solvent, carrying out a halogenation reaction of (I-4) with a halogenation reagent to give (I-5): 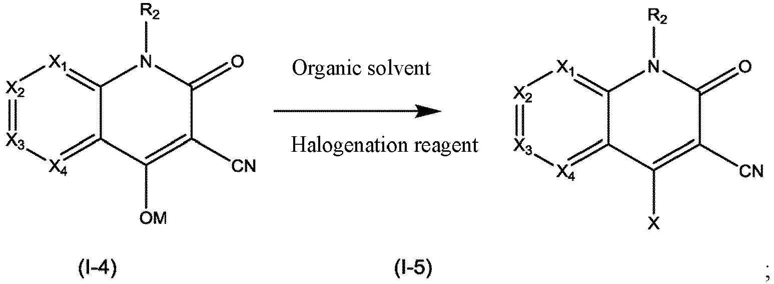
step (D): in an organic solvent, carrying out a nucleophilic substitution reaction of (I-5) with (I-6) and a base to give (I): 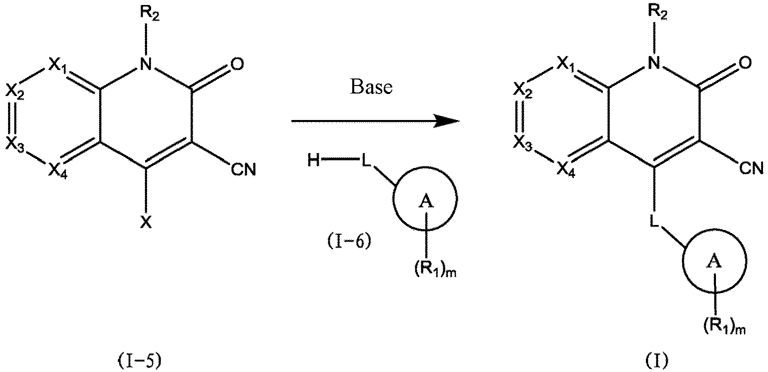
wherein X is a halogen atom; M is an alkali metal ion;
X₁ is CH, X₂ is N, X₃ is CR₃, and X₄ is CH;
R₃ is selected from hydrogen, amino, cyano, halogen, carboxyl, C₁₋₄ alkyl, C₁₋₄ alkoxy, C₁₋₄ alkylamino, (C₁₋₄ alkyl)₂amino, morpholinyl, C₂₋₆ alkenyl, C₁₋₄ alkylcarbonyl, C₁₋₄ alkylaminocarbonyl, (C₁₋₆ alkyl)₂aminocarbonyl, C₁₋₄ alkylsulfonyl, C₁₋₄ alkylthio, aminocarbonyl, cyclopropyl, azetidinyl and piperazinyl,
   a) wherein the C₁₋₄ alkyl, C₁₋₄ alkoxy, C₁₋₄ alkylamino, (C₁₋₄ alkyl)₂amino, C₂₋₆ alkenyl, C₁₋₄ alkylcarbonyl, C₁₋₄ alkylaminocarbonyl, (C₁₋₆ alkyl)₂aminocarbonyl, C₁₋₄ alkylsulfonyl, C₁₋₄ alkylthio and aminocarbonyl are unsubstituted or optionally substituted with one or more (e.g., 1, 2, 3, 4 or 5, provided that a valence balance is achieved) groups independently selected from hydroxyl, amino, halogen, C₁₋₄ alkyl, C₁₋₄ alkoxy, C₁₋₄ alkylamino, (C₁₋₄ alkyl)₂amino, cyclopropyl, C₁₋₄ alkylcarbonyloxy, and 4-6 membered heterocyclyl which is unsubstituted or substituted with C₁₋₄ alkyl; and
   b) wherein the cyclopropyl, azetidinyl, morpholinyl and piperazinyl are unsubstituted or optionally substituted with one or more (e.g., 1, 2, 3, 4 or 5, provided that a valence balance is achieved) groups independently selected from hydroxyl, halogen, C₁₋₄ alkyl, C₁₋₄ alkoxy, cyclopropyl, amino, C₁₋₄ alkylamino, (C₁₋₄ alkyl)₂amino and C₁₋₄ alkylcarbonyloxy;
L is a bond;
ring A is 4-7 membered monocyclic heterocyclyl or 7-12 membered spiro-heterocyclyl, wherein a heteroatom of the 4-7 membered monocyclic heterocyclyl is selected from N; a heteroatom of the 7-12 membered spiro-heterocyclyl is selected from one of or a combination of two of O and N; the 7-12 membered spiro-heterocyclyl contains at least one N, and ring A is linked to L by the N atom;
each R₁ is independently selected from hydrogen, hydroxyl, cyano, halogen, C₁₋₄ alkyl, C₁₋₄ alkoxy, pyrazolyl, thiazolyl and triazolyl, wherein the C₁₋₄ alkyl, C₁₋₄ alkoxy, pyrazolyl, thiazolyl and triazolyl are unsubstituted or substituted with hydroxyl;
m is 0, 1 or 2;
R₂ is selected from hydrogen.

Technical solution 2. The preparation method according to any one of the preceding technical solutions, wherein
R₃ is selected from hydrogen, halogen, C₁₋₄ alkyl, C₁₋₄ alkoxy, morpholinyl, C₂₋₆ alkenyl, C₁₋₄ alkylaminocarbonyl, (C₁₋₄ alkyl)₂aminocarbonyl and aminocarbonyl,
   a) wherein the C₁₋₄ alkyl, C₁₋₄ alkoxy, C₂₋₆ alkenyl, C₁₋₄ alkylaminocarbonyl, (C₁₋₄ alkyl)₂aminocarbonyl, and aminocarbonyl are unsubstituted or optionally substituted with one or more (e.g., 1, 2, 3, 4 or 5, provided that a valence balance is achieved) groups independently selected from hydroxyl, C₁₋₄ alkoxy, cyclopropyl, amino, C₁₋₄ alkylamino, (C₁₋₄ alkyl)₂amino, and 4-6 membered heterocyclyl which is unsubstituted or substituted with C₁₋₄ alkyl; and
   b) wherein the morpholinyl is unsubstituted or optionally substituted with one or more (e.g. 1, 2, 3, 4 or 5, provided that a valence balance is achieved) groups independently selected from hydroxyl, C₁₋₄ alkoxy, cyclopropyl, amino, C₁₋₄ alkylamino and (C₁₋₄ alkyl)₂amino;
Technical solution 3. The preparation method according to any one of the preceding technical solutions, wherein
R₃ is selected from hydrogen, halogen, C₁₋₄ alkyl, C₁₋₄ alkoxy, C₂₋₆ alkenyl, C₁₋₄ alkylaminocarbonyl and aminocarbonyl,
wherein the C₁₋₄ alkyl, C₁₋₄ alkoxy, C₂₋₆ alkenyl, C₁₋₄ alkylaminocarbonyl, and aminocarbonyl are unsubstituted or optionally substituted with one or more (e.g., 1, 2, 3, 4 or 5, provided that a valence balance is achieved) groups independently selected from hydroxyl, C₁₋₄ alkoxy, cyclopropyl, amino, C₁₋₄ alkylamino, (C₁₋₄ alkyl)₂amino, and 4-6 membered heterocyclyl which is unsubstituted or substituted with C₁₋₄ alkyl.

Technical solution 4. The preparation method according to any one of the preceding technical solutions, wherein
ring A is a 4-7 membered monocyclic heterocyclyl, wherein a heteroatom of the 4-7 membered monocyclic heterocyclyl is selected from N, and ring A is linked to L by the N atom.
Technical solution 5. The preparation method according to any one of the preceding technical solutions, wherein ring A is selected from 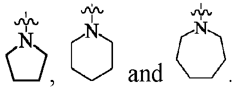
Technical solution 6. The preparation method according to any one of the preceding technical solutions, wherein ring A is 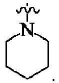
Technical solution 7. The preparation method according to any one of the preceding technical solutions, wherein
each R₁ is independently selected from hydrogen, C₁₋₄ alkyl, C₁₋₄ alkoxy, pyrazolyl, thiazolyl and triazolyl, wherein the C₁₋₄ alkyl, C₁₋₄ alkoxy, pyrazolyl, thiazolyl and triazolyl are unsubstituted or substituted with hydroxyl.

Technical solution 8. The preparation method according to any one of the preceding technical solutions, wherein
in step (C'), the halogenation reagent is one or more of phosphorus oxychloride, thionyl chloride, sulfuryl chloride, phosphorus trichloride, phosphorus pentachloride, phosphorus tribromide, phosphorus oxybromide, triphosgene and oxalyl chloride.

Technical solution 9. The preparation method according to any one of the preceding technical solutions, wherein
in step (C'), the halogenation reagent is phosphorus oxychloride.

Technical solution 10. The preparation method according to any one of the preceding technical solutions, wherein
in step (C'), a molar ratio of (I-4) to the halogenation reagent is 1:(1-5).

Technical solution 11. The preparation method according to any one of the preceding technical solutions, wherein
in step (C'), a molar ratio of (I-4) to the halogenation reagent is 1:(1-3).

Technical solution 12. The preparation method according to any one of the preceding technical solutions, wherein
in step (C'), a molar ratio of (I-4) to the halogenation reagent is 1 :(1.2-2.3).

Technical solution 13. The preparation method according to any one of the preceding technical solutions, wherein
in step (C'), a molar ratio of (I-4) to the halogenation reagent is about 1:1.5.

Technical solution 14. The preparation method according to any one of the preceding technical solutions, wherein
in step (D'), the base is one or more of triethylamine, N,N-diisopropylethylamine, sodium methoxide, sodium ethoxide, sodium tert-butoxide and potassium tert-butoxide.

Technical solution 15. The preparation method according to any one of the preceding technical solutions, wherein
in step (D'), the base is triethylamine or N,N-diisopropylethylamine.

Technical solution 16. The preparation method according to any one of the preceding technical solutions, wherein
in step (D'), the base is triethylamine.

Technical solution 17. The preparation method according to any one of the preceding technical solutions, wherein
in step (D'), the base is N,N-diisopropylethylamine.

Technical solution 18. The preparation method according to any one of the preceding technical solutions, wherein
in step (D'), a molar ratio of (I-5) to (I-6) to the base is 1:(0.5-2):(1-3).

Technical solution 19. The preparation method according to any one of the preceding technical solutions, wherein
in step (C'), the organic solvent is one or more of acetonitrile, tetrahydrofuran, 2-methyltetrahydrofuran, chloroform and 1,2-dichloroethane.

Technical solution 20. The preparation method according to any one of the preceding technical solutions, wherein
in step (C'), the organic solvent is acetonitrile.

Technical solution 21. The preparation method according to any one of the preceding technical solutions, wherein
in step (D'), the organic solvent is one or more of ethanol, tetrahydrofuran, 2-methyltetrahydrofuran, N,N-dimethylformamide and N,N-dimethylacetamide.

Technical solution 22. The preparation method according to any one of the preceding technical solutions, wherein
in step (D'), the organic solvent is ethanol.

Technical solution 23. The preparation method according to any one of the preceding technical solutions, wherein
in step (C'), the organic solvent is used in an amount that is 2-20 times the volume of formula (I'-4).

Technical solution 24. The preparation method according to any one of the preceding technical solutions, wherein
in step (C'), the organic solvent is used in an amount that is 4-10 times the volume of formula (I'-4).

Technical solution 25. The preparation method according to any one of the preceding technical solutions, wherein
in step (D'), the organic solvent is used in an amount that is 2-20 times the volume of formula (I'-5).

Technical solution 26. The preparation method according to any one of the preceding technical solutions, wherein
in step (D'), the organic solvent is used in an amount that is 10-20 times the volume of formula (I'-5).

Technical solution 27. The preparation method according to any one of the preceding technical solutions, wherein
in step (C'), the reaction temperature is 40-160 °C and is not higher than a temperature that is 5 °C lower than the boiling point of the solvent, and the reaction time is 1-36 h.

Technical solution 28. The preparation method according to any one of the preceding technical solutions, wherein
in step (C'), the reaction temperature is 60-140°C and is not higher than a temperature that is 5 °C lower than the boiling point of the solvent, and the reaction time is 2-24 h.

Technical solution 29. The preparation method according to any one of the preceding technical solutions, wherein
in step (C'), the reaction temperature is 80-120°C and is not higher than a temperature that is 5 °C lower than the boiling point of the solvent, and the reaction time is 4-12 h.

Technical solution 30. The preparation method according to any one of the preceding technical solutions, wherein
in step (D'), the reaction temperature is 40-120 °C and is not higher than a temperature that is 5 °C lower than the boiling point of the solvent, and the reaction time is 0.1-12 h.

Technical solution 31. The preparation method according to any one of the preceding technical solutions, wherein
in step (D'), the reaction temperature is 50-110°C and is not higher than a temperature that is 5 °C lower than the boiling point of the solvent, and the reaction time is 0.5-6 h.

Technical solution 32. The preparation method according to any one of the preceding technical solutions, wherein
in step (D'), the reaction temperature is 60-100°C and is not higher than a temperature that is 5 °C lower than the boiling point of the solvent, and the reaction time is 1-3 h.

Technical solution 33. A preparation method for a compound of formula (I'), comprising the following steps:

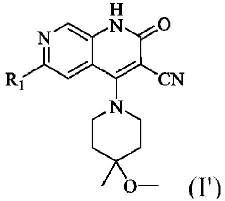

step (C): in an organic solvent, carrying out a halogenation reaction of (I'-4) with a halogenation reagent to give (I'-5): 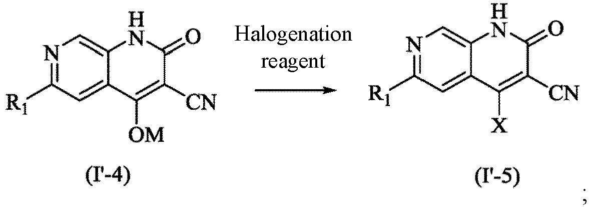
step (D): in an organic solvent, carrying out a nucleophilic substitution reaction of (I'-5) with (I'-6) and a base to give (I'): 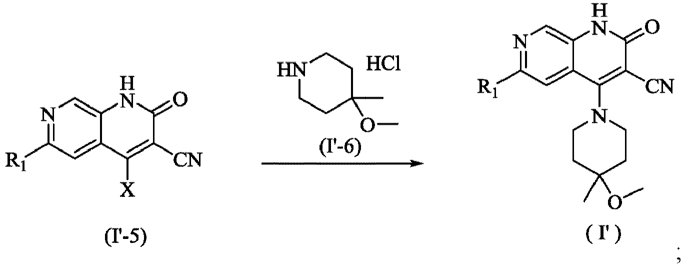
wherein R₁ is C₁₋₆ alkyl;
X is a halogen atom;
M is an alkali metal ion.

Technical solution 34. The preparation method according to technical solution 33, wherein in step (C), the halogenation reagent is one or more of phosphorus oxychloride, thionyl chloride, sulfuryl chloride, phosphorus trichloride, phosphorus pentachloride, phosphorus tribromide, phosphorus oxybromide, triphosgene and oxalyl chloride.

Technical solution 35. The preparation method according to any one of claims 33-34, wherein in step (C), the halogenation reagent is phosphorus oxychloride.

Technical solution 36. The preparation method according to any one of technical solutions 33-35, wherein in step (C), a molar ratio of (I'-4) to the halogenation reagent is 1:(1-5).

Technical solution 37. The preparation method according to any one of technical solutions 33-36, wherein in step (C), a molar ratio of (I'-4) to the halogenation reagent is 1:(1-3).

Technical solution 38. The preparation method according to any one of technical solutions 33-37, wherein in step (C), a molar ratio of (I'-4) to the halogenation reagent is 1:(1.2-2.3).

Technical solution 39. The preparation method according to any one of technical solutions 33-38, wherein in step (C), a molar ratio of (I'-4) to the halogenation reagent is about 1:1.5.

Technical solution 40. The preparation method according to any one of technical solutions 33-39, wherein in step (D), the base is one or more of triethylamine, N,N-diisopropylethylamine, sodium methoxide, sodium ethoxide, sodium tert-butoxide and potassium tert-butoxide.

Technical solution 41. The preparation method according to any one of technical solutions 33-40, wherein in step (D), the base is triethylamine or N,N-diisopropylethylamine.

Technical solution 42. The preparation method according to any one of technical solutions 33-41, wherein in step (D), the base is triethylamine.

Technical solution 43. The preparation method according to any one of technical solutions 33-42, wherein in step (D), the base is N,N-diisopropylethylamine.

Technical solution 44. The preparation method according to any one of technical solutions 33-43, wherein in step (D), a molar ratio of (I'-5) to (I'-6) to the base is 1:(0.5-2):(1-3).

Technical solution 45. The preparation method according to any one of technical solutions 33-44, wherein in step (C), the organic solvent is one or more of acetonitrile, tetrahydrofuran, 2-methyltetrahydrofuran, chloroform and 1,2-dichloroethane.

Technical solution 46. The preparation method according to any one of claims 33-45, wherein in step (C), the organic solvent is acetonitrile.

Technical solution 47. The preparation method according to any one of technical solutions 33-46, wherein in step (D), the organic solvent is one or more of ethanol, tetrahydrofuran, 2-methyltetrahydrofuran, N,N-dimethylformamide and N,N-dimethylacetamide.

Technical solution 48. The preparation method according to any one of technical solutions 33-47, wherein in step (D), the organic solvent is ethanol.

Technical solution 49. The preparation method according to any one of technical solutions 33-48, wherein in step (C), the organic solvent is used in an amount that is 2-20 times the volume of formula (I'-4).

Technical solution 50. The preparation method according to any one of technical solutions 33-49, wherein in step (C), the organic solvent is used in an amount that is 4-10 times the volume of formula (I'-4).

Technical solution 51. The preparation method according to any one of technical solutions 33-50, wherein in step (D), the organic solvent is used in an amount that is 2-20 times the volume of formula (I'-5).

Technical solution 52. The preparation method according to any one of technical solutions 33-51, wherein in step (D), the organic solvent is used in an amount that is 10-20 times the volume of formula (I'-5).

Technical solution 53. The preparation method according to any one of technical solutions 33-52, wherein in step (C), the reaction temperature is 40-160 °C and is not higher than a temperature that is 5 °C lower than the boiling point of the solvent, and the reaction time is 1-36 h.

Technical solution 54. The preparation method according to any one of technical solutions 33-53, wherein in step (C), the reaction temperature is 60-140°C and is not higher than a temperature that is 5 °C lower than the boiling point of the solvent, and the reaction time is 2-24 h.

Technical solution 55. The preparation method according to any one of technical solutions 33-54, wherein in step (C), the reaction temperature is 80-120°C and is not higher than a temperature that is 5 °C lower than the boiling point of the solvent, and the reaction time is 4-12 h.

Technical solution 56. The preparation method according to any one of technical solutions 33-55, wherein in step (D), the reaction temperature is 40-120 °C and is not higher than a temperature that is 5 °C lower than the boiling point of the solvent, and the reaction time is 0.1-12 h.

Technical solution 57. The preparation method according to any one of technical solutions 33-56, wherein in step (D), the reaction temperature is 50-110°C and is not higher than a temperature that is 5 °C lower than the boiling point of the solvent, and the reaction time is 0.5-6 h.

Technical solution 58. The preparation method according to any one of technical solutions 33-57, wherein in step (D), the reaction temperature is 60-100°C and is not higher than a temperature that is 5 °C lower than the boiling point of the solvent, and the reaction time is 1-3 h.

Technical solution 59. The preparation method according to any one of technical solutions 1-58, further comprising step (B):
in an organic solvent, carrying out a condensation reaction of (I'-3) with a base to give (I'-4): 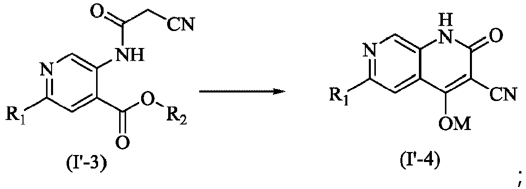
wherein R₁ is C₁₋₆ alkyl;
R₂ is C₁₋₆ alkyl;
M is K⁺, Na⁺, Li⁺ or Cs⁺.

Technical solution 60. The preparation method according to technical solution 59, wherein R₁ is methyl or ethyl.

Technical solution 61. The preparation method according to any one of technical solutions 59-60, wherein R₂ is methyl or ethyl.

Technical solution 62. The preparation method according to any one of technical solutions 59-61, wherein M is K⁺ or Na⁺.

Technical solution 63. The preparation method according to any one of technical solutions 59-62, wherein in step (B), the base is one or more of potassium hydroxide, sodium hydroxide, sodium methoxide, sodium ethoxide, sodium tert-butoxide, potassium tert-butoxide and potassium methoxide.

Technical solution 64. The preparation method according to any one of technical solutions 59-63, wherein in step (B), the base is potassium methoxide and/or sodium ethoxide.

Technical solution 65. The preparation method according to any one of technical solutions 59-64, wherein in step (B), a molar ratio of (I'-3) to the base is 1:(1-5).

Technical solution 66. The preparation method according to any one of technical solutions 59-65, wherein in step (B), the organic solvent is one or more of ethanol, tetrahydrofuran, 2-methyltetrahydrofuran, N,N-dimethylformamide and N,N-dimethylacetamide.

Technical solution 67. The preparation method according to any one of technical solutions 59-66, wherein in step (B), the organic solvent is tetrahydrofuran.

Technical solution 68. The preparation method according to any one of technical solutions 59-67, wherein in step (B), the organic solvent is used in an amount that is 2-20 times the volume of formula (I'-3).

Technical solution 69. The preparation method according to any one of technical solutions 59-68, wherein in step (B), the organic solvent is used in an amount that is 3-12 times the volume of formula (I'-3).

Technical solution 70. The preparation method according to any one of technical solutions 59-69, wherein in step (B), the reaction temperature is 40-120 °C and is not higher than a temperature that is 5 °C lower than the boiling point of the solvent, and the reaction time is 0.1-12 h.

Technical solution 71. The preparation method according to any one of technical solutions 59-70, wherein in step (B), the reaction temperature is 50-120 °C and is not higher than a temperature that is 5 °C lower than the boiling point of the solvent, and the reaction time is 0.5-6 h.

Technical solution 72. The preparation method according to any one of technical solutions 59-71, wherein in step (B), the reaction temperature is 60-100°C and is not higher than a temperature that is 5 °C lower than the boiling point of the solvent, and the reaction time is 1-3 h.

Technical solution 73. The preparation method according to any one of technical solutions 59-72, further comprising step (A):
in an organic solvent, carrying out a condensation reaction of formulas (I'-1) and (I'-2) with a coupling reagent to give (I'-3): 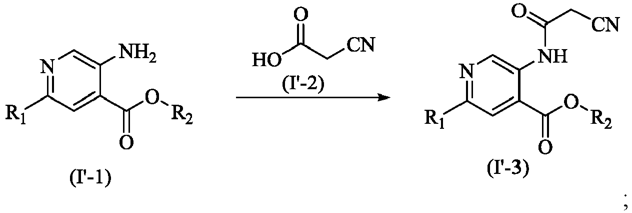
wherein R₁ is C₁₋₆ alkyl;
R₂ is C₁₋₆ alkyl.

Technical solution 74. The preparation method according to technical solution 73, wherein R₁ is methyl or ethyl.

Technical solution 75. The preparation method according to any one of technical solutions 73-74, wherein R₂ is methyl or ethyl.

Technical solution 76. The preparation method according to any one of technical solutions 73-75, wherein in step (A), the coupling reagent is one or more of 1-(3-dimethylaminopropyl)-3-ethylcarbodiimide hydrochloride, 2-(7-oxidobenzotriazole)-N,N,N',N'-tetramethyluronium hexafluorophosphate, benzotriazole-1-yloxytris(dimethylamino)phosphonium hexafluorophosphate and dicyclohexylcarbodiimide.

Technical solution 77. The preparation method according to any one of technical solutions 73-76, wherein in step (A), the coupling reagent is 1-(3-dimethylaminopropyl)-3-ethylcarbodiimide hydrochloride.

Technical solution 78. The preparation method according to any one of technical solutions 73-77, wherein in step (A), a molar ratio of (I'-1) to (I'-2) to the coupling reagent is 1:(0.5-2):(1-2).

Technical solution 79. The preparation method according to any one of technical solutions 73-78, wherein in step (A), the organic solvent is one or more of dichloromethane, N,N-dimethylformamide, N,N-dimethylacetamide and tetrahydrofuran.

Technical solution 80. The preparation method according to any one of technical solutions 73-79, wherein in step (A), the organic solvent is dichloromethane.

Technical solution 81. The preparation method according to any one of technical solutions 73-80, wherein in step (A), the organic solvent is used in an amount that is 2-20 times the volume of formula (I'-1).

Technical solution 82. The preparation method according to any one of technical solutions 73-81, wherein in step (A), the organic solvent is used in an amount that is 5-12 times the volume of formula (I'-1).

Technical solution 83. The preparation method according to any one of technical solutions 73-82, wherein in step (A), the reaction temperature is 10-40 °C and is not higher than a temperature that is 5 °C lower than the boiling point of the solvent, and the reaction time is 0.1-12 h.

Technical solution 84. The preparation method according to any one of technical solutions 73-83, wherein in step (A), the reaction temperature is 15-35°C and is not higher than a temperature that is 5 °C lower than the boiling point of the solvent, and the reaction time is 0.5-6 h.

Technical solution 85. The preparation method according to any one of technical solutions 73-84, wherein in step (A), the reaction temperature is 20-30°C and is not higher than a temperature that is 5 °C lower than the boiling point of the solvent, and the reaction time is 1-3 h.

Technical solution 86. An intermediate for a compound of formula (I'), having the following structural formula: 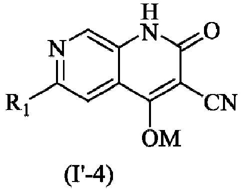
wherein R₁ is C₁₋₆ alkyl;
M is K⁺, Na⁺, Li⁺ or Cs⁺.

Technical solution 87. An intermediate for a compound of formula (I'), having the following structural formula: 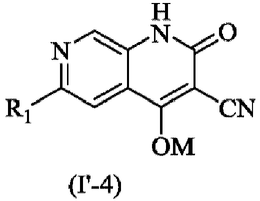
wherein R₁ is methyl or ethyl;
M is K⁺, Na⁺, Li⁺ or Cs⁺.

Technical solution 88. An intermediate, having the following structure: 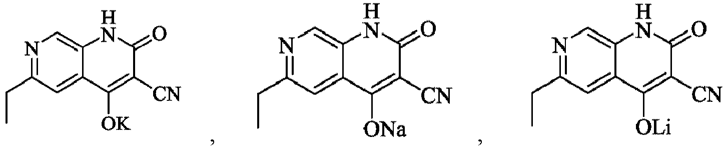 or

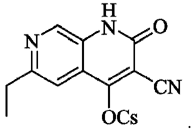

The preparation methods disclosed in WO2019062733A1 and WO2020182076A1 use a class of 1,2-dihydro-2-oxo-3-cyano-1,7-naphthyridin-4-ol compounds as intermediates for halogenation with a halogenation reagent; in contrast, according to the preparation method of the present invention, a class of alkali metal salt compounds of 1,2-dihydro-2-oxo-3-cyano-1,7-naphthyridin-4-ol can be used as intermediates for halogenation with a halogenation reagent. As illustrated in the examples below, the present invention allows for a reduction in the use of toxic and hazardous halogenation reagents (such as phosphorus oxychloride).

### Examples

The above description of the present invention is explained in further detail below by specific embodiments, but the scope of the present invention should not be construed as being limited to the following examples. All techniques implemented based on the above description of the present invention fall within the scope of the present invention.

### Abbreviations used herein:

"EDCI": 1-(3-dimethylaminopropyl)-3-ethylcarbodiimide hydrochloride.

"DIPEA": N,N-diisopropylethylamine. 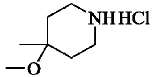 was prepared according to the method for preparing compound 23 in Example 14 of WO2009093032A1. Example 1: Preparation of compound of formula (I)

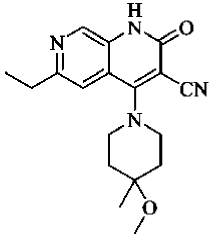

### Step 1a: Synthesis of ethyl 6-ethyl-3-(cyanoacetamido)pyridine-4-carboxylate

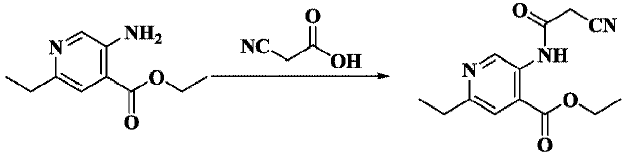

The intermediate ethyl 6-ethyl-3-aminopyridine-4-carboxylate (100 g, 514.84 mmol, 1.0 eq) was dissolved in dichloromethane (1.00 L). Cyanoacetic acid (52.55 g, 617.81 mmol, 1.2 eq) was added under ice bath conditions, and EDCI (148.03 g, 772.26 mmol, 1.5 eq) was added portionwise. The mixture was left to react at 25 °C for 2 h. LC-MS analysis showed the reaction was complete. H₂O (1.00 L) was added to the reaction solution. The organic phase was separated, washed with H₂O (2 × 500 mL), dried over anhydrous sodium sulfate, and filtered under vacuum. The filtrate was concentrated, and the crude product was slurried with methyl tert-butyl ether (300 mL) to give the product (128 g, yield: 95.15%).

### Step 1b: Synthesis of ethyl 6-ethyl-3-(cyanoacetamido)pyridine-4-carboxylate

The intermediate ethyl 6-ethyl-3-aminopyridine-4-carboxylate (500 g, 2.57 mol, 1.0 eq) was dissolved in dichloromethane (6.00 L). Cyanoacetic acid (262.76 g, 3.09 mol, 1.2 eq) was added under ice bath conditions, and EDCI (740.23 g, 3.86 mol, 1.5 eq) was added portionwise. The mixture was left to react at 25 °C for 2 h. LC-MS analysis showed the reaction was complete. H₂O (5.00 L) was added to the reaction solution. The aqueous phase was separated and extracted with DCM (2 × 500 mL). The organic phases were combined, dried over anhydrous sodium sulfate, and filtered under vacuum. The filtrate was concentrated to 1.0 L and added dropwise to 5.0 L of n-heptane, and a large amount of solid precipitated. The solid was collected by filtration under vacuum and dried at 50 °C to give the product (658 g, yield: 98%).

### Step 2a: Synthesis of potassium 3-cyano-6-ethyl-2-oxo-1,2-dihydro-1,7-naphthyri din-4-olate

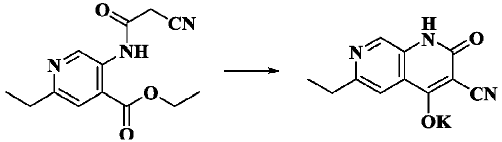

Potassium methoxide (2.95 g, 42.10 mmol, 1.1 eq) was dispersed in n-heptane (200 mL), and the internal temperature of the system was then raised to 80 °C. Subsequently, the intermediate ethyl 6-ethyl-3-(cyanoacetamido)pyridine-4-carboxylate (10 g, 38.27 mmol, 1.0 eq) was dissolved in tetrahydrofuran (30 mL), and the solution was added dropwise to the above system being heated. After the addition, the mixture was left to react at 80 °C for 2 h. LC-MS analysis showed the reaction was complete. The heating was stopped, and the reaction system was allowed to cool naturally to room temperature. The reaction solution was filtered under vacuum, and the filter cake was dried to give a crude product (10.4 g, yield: 107.29%).

### Step 2b: Synthesis of sodium 3-cyano-6-ethyl-2-oxo-1,2-dihydro-1,7-naphthyridin-4-olate

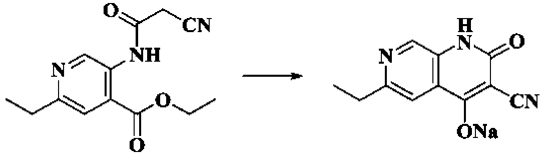

Sodium ethoxide (2.86 g, 42.10 mmol, 1.1 eq) was dispersed in n-heptane (200 mL), and the internal temperature of the system was then raised to 80 °C. Subsequently, the intermediate ethyl 6-ethyl-3-(cyanoacetamido)pyridine-4-carboxylate (10 g, 38.27 mmol, 1.0 eq) was dissolved in tetrahydrofuran (30 mL), and the solution was added dropwise to the above system being heated. After the addition, the mixture was left to react at 80 °C for 2 h. LC-MS analysis showed the reaction was complete. The heating was stopped, and the reaction system was allowed to cool naturally to room temperature. The reaction solution was filtered under vacuum, and the filter cake was dried to give a crude product (10.3 g, yield: 113.43%).

### Step 3a: Synthesis of 6-ethyl-4-chloro-2-oxo-1,2-dihydro-1,7-naphthyridine-3-carbonitrile

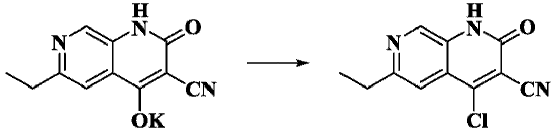

The intermediate potassium 3-cyano-6-ethyl-2-oxo-1,2-dihydro-1,7-naphthyridin-4-olate (5 g, 19.74 mmol, 1.0 eq) was dissolved in acetonitrile (50 mL), and phosphorus oxychloride (4.54 g, 29.61 mmol, 1.5 eq) was added at room temperature. The mixture was left to react at 100 °C for 8 h. The reaction solution was cooled and concentrated, and H₂O (50 mL) was added. The pH was adjusted to about 6 with 10% sodium hydroxide, and a large amount of yellow solid precipitated. The solid was collected by filtration under vacuum and dried to give a crude product (3.87 g, yield: 83.95%).

### Step 3b: Synthesis of 6-ethyl-4-chloro-2-oxo-1,2-dihydro-1,7-naphthyridine-3-carbonitrile

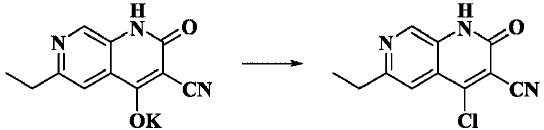

The intermediate potassium 3-cyano-6-ethyl-2-oxo-1,2-dihydro-1,7-naphthyridin-4-olate (5 g, 19.74 mmol, 1.0 eq) was dissolved in acetonitrile (50 mL), and phosphorus oxychloride (3.63 g, 23.69 mmol, 1.2 eq) was added at room temperature. The mixture was left to react at 100 °C for 8 h. The reaction solution was cooled and concentrated, and H₂O (50 mL) was added. The pH was adjusted to about 6 with 10% sodium hydroxide, and a large amount of yellow solid precipitated. The solid was collected by filtration under vacuum and dried to give a crude product (3.47 g, yield: 75.27%).

### Step 3c: Synthesis of 6-ethyl-4-chloro-2-oxo-1,2-dihydro-1,7-naphthyridine-3-carbonitrile

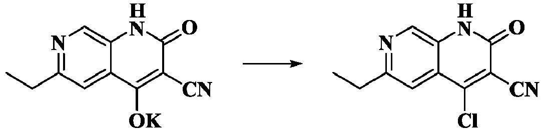

The intermediate potassium 3-cyano-6-ethyl-2-oxo-1,2-dihydro-1,7-naphthyridin-4-olate (10 g, 39.5 mmol, 1.0 eq) was dissolved in acetonitrile (100 mL), and phosphorus oxychloride (13.93 g, 90.85 mmol, 2.3 eq) was added portionwise at room temperature. The mixture was left to react at 100 °C for 8 h. The reaction solution was cooled and concentrated, and H₂O (500 mL) was added. The pH was adjusted to about 6 with 10% sodium hydroxide, and a large amount of yellow solid precipitated. The solid was collected by filtration under vacuum and dried to give a crude product (7.18 g, yield: 77.87%).

### Step 3d: Synthesis of 6-ethyl-4-chloro-2-oxo-1,2-dihydro-1,7-naphthyridine-3-carbonitrile

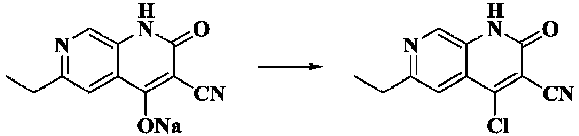

The intermediate sodium 3-cyano-6-ethyl-2-oxo-1,2-dihydro-1,7-naphthyridin-4-olate (5 g, 21.08 mmol, 1.0 eq) was dissolved in acetonitrile (50 mL), and phosphorus oxychloride (4.85 g, 31.62 mmol, 1.5 eq) was added at room temperature. The mixture was left to react at 100 °C for 8 h. The reaction solution was cooled and concentrated, and H₂O (50 mL) was added. The pH was adjusted to about 6 with 10% sodium hydroxide, and a large amount of yellow solid precipitated. The solid was collected by filtration under vacuum and dried to give a crude product (3.72 g, yield: 75.46%).

### Step 3e: Synthesis of 6-ethyl-4-chloro-2-oxo-1,2-dihydro-1,7-naphthyridine-3-carbonitrile

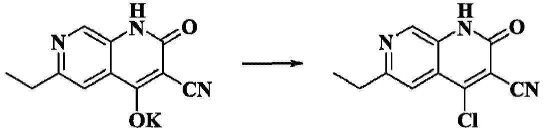

The intermediate potassium 3-cyano-6-ethyl-2-oxo-1,2-dihydro-1,7-naphthyridin-4-olate (120 g, 473.7 mmol, 1.0 eq) was dissolved in acetonitrile (1200 mL), and phosphorus oxychloride (73.36 g, 478.48 mmol, 1.01 eq) was added portionwise at room temperature. The mixture was left to react at 100 °C for 8 h. The reaction solution was cooled and concentrated, and H₂O (1200 mL) was added. The pH was adjusted to about 6 with 10% sodium hydroxide, and a large amount of yellow solid precipitated. The solid was collected by filtration under vacuum and dried to give a crude product. 2350 mL of tetrahydrofuran (THF) was added to the crude product, and activated carbon was added. The mixture was heated at reflux and filtered. The filtrate was evaporated with isopropyl acetate to remove THF and cooled to room temperature, and a large amount of solid precipitated. The solid was collected and dried to give a yellow solid (80.45 g, yield: 72.7%).

### Step 4a: Synthesis of 6-ethyl-4-(4-methoxy-4-methylpiperidin-1-yl)-2-oxo-1,2-dihydro-1,7-naphthyridine-3-carbonitrile

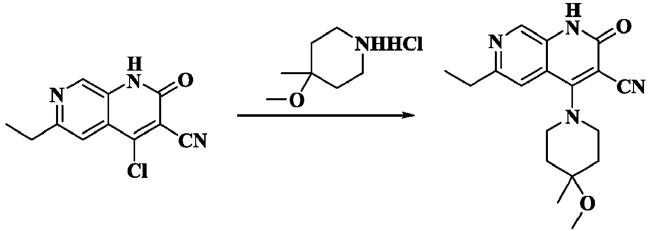

The intermediate 6-ethyl-4-chloro-2-oxo-1,2-dihydro-1,7-naphthyridine-3-carbonitrile (91 g, 390.3 mmol, 1.0 eq) and 4-methyl-4-methoxypiperidine hydrochloride (70.89 g, 429.43 mmol, 1.1 eq) were dissolved in ethanol (1365 mL), and DIPEA (151.36 g, 1171.17 mmol, 3.0 eq) was added. The mixture was left to react at 80 °C for 2 h. LC-MS analysis showed the reaction was complete. About 75% of ethanol was removed by evaporation under reduced pressure. Water (2 L) was added, and the mixture was stirred at 25 °C for 1 h and filtered under vacuum. The filter cake was refluxed with ethanol (4 L) until it was completely dissolved, and the mixture was filtered under vacuum. The filtrate was concentrated to about 1 L, cooled to about 10 °C and filtered under vacuum. The filter cake was dried to give the product (69 g, yield: 54.15%).

¹HNMR (400 MHz, DMSO-*d₆*) δ (ppm): 11.90 (s, 1H), 8.58 (s, 1H), 7.40 (s, 1H), 3.59-3.61 (m, 4H), 3.19 (s, 3H), 2.78-2.84 (m, 2H), 1.89-1.93 (m, 2H), 1.77-1.82 (m, 2H), 1.22-1.26 (m, 6H).

Molecular formula: C₁₈H₂₂N₄O₂; molecular weight: 326.40 LC-MS (Pos, *m*/*z*) = 327.59[M+H]⁺.

### Step 4b: Synthesis of 6-ethyl-4-(4-methoxy-4-methylpiperidin-1-yl)-2-oxo-1,2-dihydro-1,7-naphthyridine-3-carbonitrile

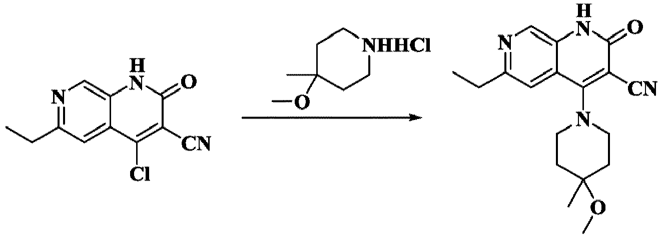

The intermediate 6-ethyl-4-chloro-2-oxo-1,2-dihydro-1,7-naphthyridine-3-carbonitrile (15 g, 64.2 mmol, 1.0 eq) and 4-methyl-4-methoxypiperidine hydrochloride (11.7 g, 70.62 mmol, 1.1 eq) were dissolved in ethanol (150 mL), and triethylamine (14.29 g, 141.23 mmol, 2.2 eq) was added. The mixture was left to react at 80 °C for 2 h. LC-MS analysis showed the reaction was complete. About 75% of ethanol was removed by evaporation under reduced pressure. Water (300 mL) was added, and the mixture was stirred at 25 °C for 1 h and filtered under vacuum. The filter cake was refluxed with ethanol (600 mL) until it was completely dissolved, and activated carbon (2 g) was added. The mixture was refluxed for 0.5 h. After filtration under vacuum, the filtrate was concentrated to about 400 mL, cooled to room temperature, and filtered under vacuum. The filter cake was dried to give the product (17.10 g, yield: 81.6%).

### Example 2

By replacing EDCI with 2-(7-oxidobenzotriazole)-N,N,N',N'-tetramethyluronium hexafluorophosphate, benzotriazole-1-yloxytris(dimethylamino)phosphonium hexafluorophosphate or dicyclohexylcarbodiimide and replacing dichloromethane with N,N-dimethylformamide, N,N-dimethylacetamide or tetrahydrofuran in step 1, ethyl 6-ethyl-3-(cyanoacetamido)pyridine-4-carboxylate can be obtained.

By replacing potassium methoxide with sodium ethoxide, potassium hydroxide, sodium hydroxide, sodium methoxide, sodium tert-butoxide or potassium tert-butoxide and replacing tetrahydrofuran with ethanol, 2-methyltetrahydrofuran, N,N-dimethylformamide or N,N-dimethylacetamide in step 2a, a corresponding product can be obtained.

By replacing sodium ethoxide with potassium methoxide, potassium hydroxide, sodium hydroxide, sodium methoxide, sodium tert-butoxide or potassium tert-butoxide and replacing tetrahydrofuran with ethanol, 2-methyltetrahydrofuran, N,N-dimethylformamide or N,N-dimethylacetamide in step 2b, a corresponding product can be obtained.

By replacing acetonitrile with tetrahydrofuran, 2-methyltetrahydrofuran, chloroform or 1,2-dichloroethane and replacing phosphorus oxychloride with thionyl chloride, sulfuryl chloride, phosphorus trichloride, phosphorus pentachloride, phosphorus tribromide, phosphorus oxybromide, triphosgene or oxalyl chloride in steps 3a, 3b, 3c and 3d, 6-ethyl-4-chloro-2-oxo-1,2-dihydro-1,7-naphthyridine-3-carbonitrile can be obtained.

By replacing N,N-diisopropylethylamine with triethylamine, sodium methoxide, sodium ethoxide, sodium tert-butoxide or potassium tert-butoxide and replacing ethanol with tetrahydrofuran, 2-methyltetrahydrofuran, N,N-dimethylformamide or N,N-dimethylacetamide in step 4a, 6-ethyl-4-(4-methoxy-4-methylpiperidin-1-yl)-2-oxo-1,2-dihydro-1,7-naphthyridine-3-carbonitrile can be obtained.

By replacing triethylamine with N,N-diisopropylethylamine, sodium methoxide, sodium ethoxide, sodium tert-butoxide or potassium tert-butoxide and replacing ethanol with tetrahydrofuran, 2-methyltetrahydrofuran, N,N-dimethylformamide or N,N-dimethylacetamide in step 4b, 6-ethyl-4-(4-methoxy-4-methylpiperidin-1-yl)-2-oxo-1,2-dihydro-1,7-naphthyridine-3-carbonitrile can be obtained.

The new preparation method of the present invention has the following advantages:
(1) Compared to the prior art, the present invention can significantly reduce the use of halogenation reagents such as phosphorus oxychloride when producing the same or even a higher yield. That is to say, less halogenation reagent such as phosphorus oxychloride can be used to obtain the same or even more amount of the desired product. Thus, the production costs are reduced, and the generation of the three types of industrial waste is significantly reduced.
(2) In massive industrial production, the heavy use of certain halogenation reagents such as phosphorus oxychloride causes the instantaneous generation of a large amount of heat during the quenching step of the workup. There is a risk of explosion if it is not properly handled. The present invention significantly reduces the use of such halogenation reagents as phosphorus oxychloride and therefore the generation of heat in the workup, thereby improving the safety of the workup system. The present invention is more suitable for use in massive industrial production.

The above description is only preferred examples of the present invention, and is not intended to limit the present invention. Any modifications, equivalents, improvements and the like made without departing from the spirit and principle of the present invention fall within the protection scope of the present invention.

## Claims

1. A preparation method for a compound of formula (I), comprising the following steps: 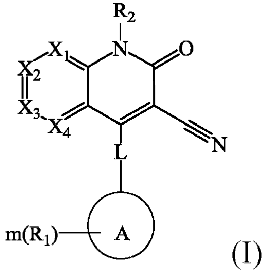
step (C'): in an organic solvent, carrying out a halogenation reaction of (I-4) with a halogenation reagent to give (I-5): 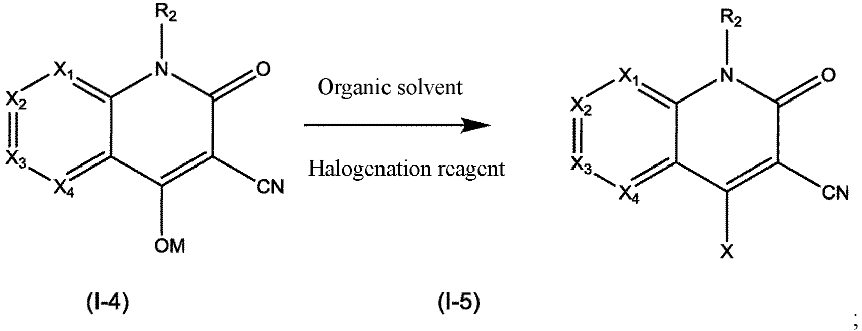
step (D'): in an organic solvent, carrying out a nucleophilic substitution reaction of (I-5) with (I-6) and a base to give (I): 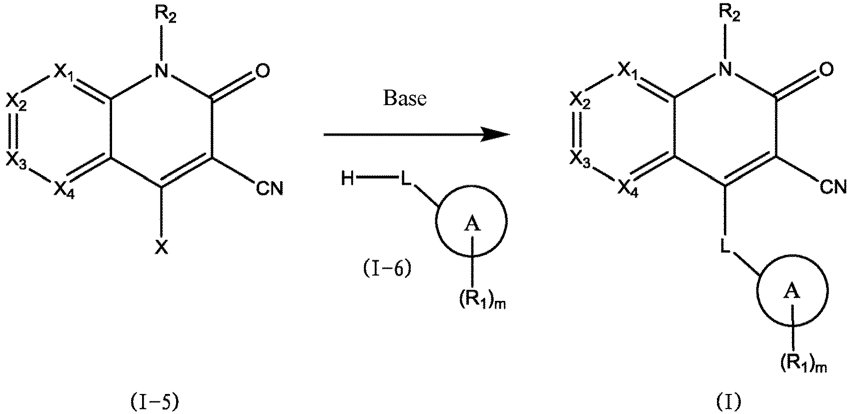
wherein X is a halogen atom; M is an alkali metal ion;
X₁ is CH, X₂ is N, X₃ is CR₃, and X₄ is CH;
R₃ is selected from hydrogen, amino, cyano, halogen, carboxyl, C₁₋₄ alkyl, C₁₋₄ alkoxy, C₁₋₄ alkylamino, (C₁₋₄ alkyl)₂amino, morpholinyl, C₂₋₆ alkenyl, C₁₋₄ alkylcarbonyl, C₁₋₄ alkylaminocarbonyl, (C₁₋₆ alkyl)₂aminocarbonyl, C₁₋₄ alkylsulfonyl, C₁₋₄ alkylthio, aminocarbonyl, cyclopropyl, azetidinyl and piperazinyl,
a) wherein the C₁₋₄ alkyl, C₁₋₄ alkoxy, C₁₋₄ alkylamino, (C₁₋₄ alkyl)₂amino, C₂₋₆ alkenyl, C₁₋₄ alkylcarbonyl, C₁₋₄ alkylaminocarbonyl, (C₁₋₆ alkyl)₂aminocarbonyl, C₁₋₄ alkylsulfonyl, C₁₋₄ alkylthio and aminocarbonyl are unsubstituted or optionally substituted with one or more (e.g., 1, 2, 3, 4 or 5, provided that a valence balance is achieved) groups independently selected from hydroxyl, amino, halogen, C₁₋₄ alkyl, C₁₋₄ alkoxy, C₁₋₄ alkylamino, (C₁₋₄ alkyl)₂amino, cyclopropyl, C₁₋₄ alkylcarbonyloxy, and 4-6 membered heterocyclyl which is unsubstituted or substituted with C₁₋₄ alkyl; and
b) wherein the cyclopropyl, azetidinyl, morpholinyl and piperazinyl are unsubstituted or optionally substituted with one or more (e.g., 1, 2, 3, 4 or 5, provided that a valence balance is achieved) groups independently selected from hydroxyl, halogen, C₁₋₄ alkyl, C₁₋₄ alkoxy, cyclopropyl, amino, C₁₋₄ alkylamino, (C₁₋₄ alkyl)₂amino and C₁₋₄ alkylcarbonyloxy;
preferably, R₃ is selected from hydrogen, halogen, C₁₋₄ alkyl, C₁₋₄ alkoxy, morpholinyl, C₂₋₆ alkenyl, C₁₋₄ alkylaminocarbonyl, (C₁₋₄ alkyl)₂aminocarbonyl, and aminocarbonyl,
a) wherein the C₁₋₄ alkyl, C₁₋₄ alkoxy, C₂₋₆ alkenyl, C₁₋₄ alkylaminocarbonyl, (C₁₋₄ alkyl)₂aminocarbonyl, and aminocarbonyl are unsubstituted or optionally substituted with one or more (e.g., 1, 2, 3, 4 or 5, provided that a valence balance is achieved) groups independently selected from hydroxyl, C₁₋₄ alkoxy, cyclopropyl, amino, C₁₋₄ alkylamino, (C₁₋₄ alkyl)₂amino, and 4-6 membered heterocyclyl which is unsubstituted or substituted with C₁₋₄ alkyl; and
b) wherein the morpholinyl is unsubstituted or optionally substituted with one or more (e.g. 1, 2, 3, 4 or 5, provided that a valence balance is achieved) groups independently selected from hydroxyl, C₁₋₄ alkoxy, cyclopropyl, amino, C₁₋₄ alkylamino and (C₁₋₄ alkyl)₂amino;
more preferably, R₃ is selected from hydrogen, halogen, C₁₋₄ alkyl, C₁₋₄ alkoxy, C₂₋₆ alkenyl, C₁₋₄ alkylaminocarbonyl and aminocarbonyl,
wherein the C₁₋₄ alkyl, C₁₋₄ alkoxy, C₂₋₆ alkenyl, C₁₋₄ alkylaminocarbonyl, and aminocarbonyl are unsubstituted or optionally substituted with one or more (e.g., 1, 2, 3, 4 or 5, provided that a valence balance is achieved) groups independently selected from hydroxyl, C₁₋₄ alkoxy, cyclopropyl, amino, C₁₋₄ alkylamino, (C₁₋₄ alkyl)₂amino, and 4-6 membered heterocyclyl which is unsubstituted or substituted with C₁₋₄ alkyl;
L is a bond;
ring A is 4-7 membered monocyclic heterocyclyl or 7-12 membered spiro-heterocyclyl, wherein a heteroatom of the 4-7 membered monocyclic heterocyclyl is selected from N; a heteroatom of the 7-12 membered spiro-heterocyclyl is selected from one of or a combination of two of O and N; the 7-12 membered spiro-heterocyclyl contains at least one N, and ring A is linked to L by the N atom;
preferably, ring A is a 4-7 membered monocyclic heterocyclyl, wherein a heteroatom of the 4-7 membered monocyclic heterocyclyl is selected from N, and ring A is linked to L by the N atom;
more preferably, ring A is selected from 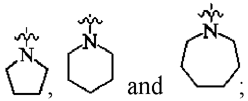
particularly more preferably, ring A is 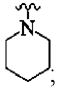
each R₁ is independently selected from hydrogen, hydroxyl, cyano, halogen, C₁₋₄ alkyl, C₁₋₄ alkoxy, pyrazolyl, thiazolyl and triazolyl, wherein the C₁₋₄ alkyl, C₁₋₄ alkoxy, pyrazolyl, thiazolyl and triazolyl are unsubstituted or substituted with hydroxyl;
preferably, each R₁ is independently selected from hydrogen, C₁₋₄ alkyl, C₁₋₄ alkoxy, pyrazolyl, thiazolyl and triazolyl, wherein the C₁₋₄ alkyl, C₁₋₄ alkoxy, pyrazolyl, thiazolyl and triazolyl are unsubstituted or substituted with hydroxyl;
m is 0, 1 or 2;
R₂ is selected from hydrogen.

2. The preparation method according to claim 1, wherein
in step (C'), the halogenation reagent is one or more of phosphorus oxychloride, thionyl chloride, sulfuryl chloride, phosphorus trichloride, phosphorus pentachloride, phosphorus tribromide, phosphorus oxybromide, triphosgene and oxalyl chloride; preferably, the halogenation reagent is phosphorus oxychloride; and/or
in step (C'), a molar ratio of (I-4) to the halogenation reagent is 1:(1-5), such as 1:(1-3), preferably 1:(1.2-2.3), and more preferably about 1:1.5; and/or
in step (D'), the base is one or more of triethylamine, N,N-diisopropylethylamine, sodium methoxide, sodium ethoxide, sodium tert-butoxide and potassium tert-butoxide; preferably, the base is triethylamine or N,N-diisopropylethylamine; and/or
in step (D'), a molar ratio of (I-5) to (I-6) to the base is 1:(0.5-2):(1-3).

3. The preparation method according to any one of claims 1-2, wherein
in step (C'), the organic solvent is one or more of acetonitrile, tetrahydrofuran, 2-methyltetrahydrofuran, chloroform and 1,2-dichloroethane; preferably acetonitrile; and/or
in step (D'), the organic solvent is one or more of ethanol, tetrahydrofuran, 2-methyltetrahydrofuran, N,N-dimethylformamide and N,N-dimethylacetamide; preferably ethanol; and/or
in step (C'), the organic solvent is used in an amount that is 2-20 times, preferably 4-10 times, the volume of formula (I'-4); and/or
in step (D'), the organic solvent is used in an amount that is 2-20 times, preferably 10-20 times, the volume of formula (I'-5).

4. A preparation method for a compound of formula (I'), comprising the following steps: 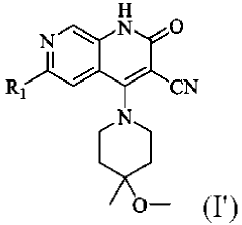
step (C): in an organic solvent, carrying out a halogenation reaction of (I'-4) with a halogenation reagent to give (I'-5): 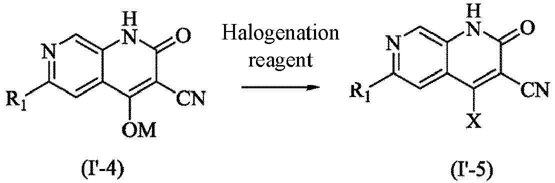
step (D): in an organic solvent, carrying out a nucleophilic substitution reaction of (I'-5) with (I'-6) and a base to give (I'): 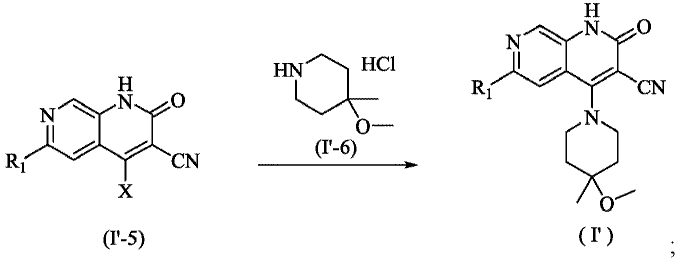
wherein R₁ is C₁₋₆ alkyl;
X is a halogen atom;
M is an alkali metal ion.

5. The preparation method according to claim 4, wherein
in step (C), the halogenation reagent is one or more of phosphorus oxychloride, thionyl chloride, sulfuryl chloride, phosphorus trichloride, phosphorus pentachloride, phosphorus tribromide, phosphorus oxybromide, triphosgene and oxalyl chloride; preferably, the halogenation reagent is phosphorus oxychloride; and/or
in step (C), a molar ratio of (I'-4) to the halogenation reagent is 1:(1-5), such as 1:(1-3), preferably 1:(1.2-2.3), and more preferably about 1:1.5; and/or
in step (D), the base is one or more of triethylamine, N,N-diisopropylethylamine, sodium methoxide, sodium ethoxide, sodium tert-butoxide and potassium tert-butoxide; preferably, the base is triethylamine or N,N-diisopropylethylamine; and/or
in step (D), a molar ratio of (I'-5) to (I'-6) to the base is 1:(0.5-2):(1-3).

6. The preparation method according to any one of claims 4-5, wherein
in step (C), the organic solvent is one or more of acetonitrile, tetrahydrofuran, 2-methyltetrahydrofuran, chloroform and 1,2-dichloroethane; preferably acetonitrile; and/or
in step (D), the organic solvent is one or more of ethanol, tetrahydrofuran, 2-methyltetrahydrofuran, N,N-dimethylformamide and N,N-dimethylacetamide; preferably ethanol; and/or
in step (C), the organic solvent is used in an amount that is 2-20 times, preferably 4-10 times, the volume of formula (I'-4); and/or
in step (D), the organic solvent is used in an amount that is 2-20 times, preferably 10-20 times, the volume of formula (I'-5).

7. The preparation method according to any one of claims 1-6, further comprising step (B):
in an organic solvent, carrying out a condensation reaction of (I'-3) with a base to give (I'-4): 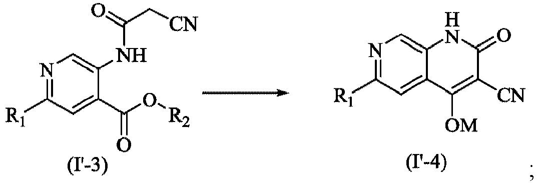
wherein R₁ is C₁₋₆ alkyl, preferably methyl or ethyl;
R₂ is C₁₋₆ alkyl, preferably methyl or ethyl;
M is K⁺, Na⁺, Li⁺ or Cs⁺, preferably K⁺ or Na⁺.

8. The preparation method according to claim 7, wherein
in step (B), the base is one or more of potassium hydroxide, sodium hydroxide, sodium methoxide, sodium ethoxide, sodium tert-butoxide, potassium tert-butoxide and potassium methoxide; preferably, the base is one or more of potassium methoxide and sodium ethoxide; and/or
in step (B), a molar ratio of (I'-3) to the base is 1:(1-5).

9. The preparation method according to any one of claims 7-8, wherein
in step (B), the organic solvent is one or more of ethanol, tetrahydrofuran, 2-methyltetrahydrofuran, N,N-dimethylformamide and N,N-dimethylacetamide, preferably tetrahydrofuran; and/or
in step (B), the organic solvent is used in an amount that is 2-20 times, preferably 3-12 times, the volume of formula (I'-3).

10. The preparation method according to any one of claims 7-9, further comprising step (A):
in an organic solvent, carrying out a condensation reaction of formulas (I'-1) and (I'-2) with a coupling reagent to give (I'-3): 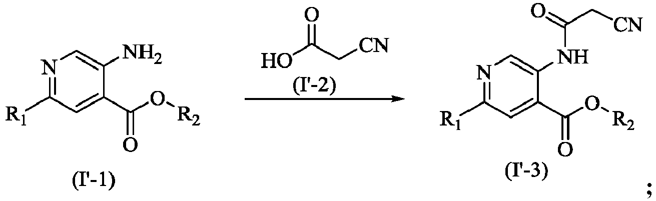
wherein R₁ is C₁₋₆ alkyl, preferably methyl or ethyl;
R₂ is C₁₋₆ alkyl, preferably methyl or ethyl.

11. The preparation method according to claim 10, wherein
in step (A), the coupling reagent is one or more of 1-(3-dimethylaminopropyl)-3-ethylcarbodiimide hydrochloride, 2-(7-oxidobenzotriazole)-N,N,N',N'-tetramethyluronium hexafluorophosphate, benzotriazole-1 -yloxytris(dimethylamino)phosphonium hexafluorophosphate and dicyclohexylcarbodiimide; preferably, the coupling reagent is 1-(3-dimethylaminopropyl)-3-ethylcarbodiimide hydrochloride; and/or
in step (A), a molar ratio of (I'-1) to (I'-2) to the coupling reagent is 1:(0.5-2):(1-2).

12. The preparation method according to any one of claims 10-11, wherein
in step (A), the organic solvent is one or more of dichloromethane, N,N-dimethylformamide, N,N-dimethylacetamide and tetrahydrofuran, preferably dichloromethane; and/or
in step (A), the organic solvent is used in an amount that is 2-20 times, preferably 5-12 times, the volume of formula (I'-1).

13. A preparation intermediate for a compound of formula (I'), having the following structural formula: 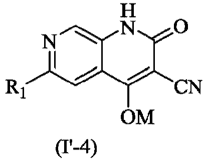
wherein R₁ is C₁₋₆ alkyl, preferably methyl or ethyl;
M is K⁺, Na⁺, Li⁺ or Cs⁺.

14. The preparation intermediate according to claim 13, having the following structure: 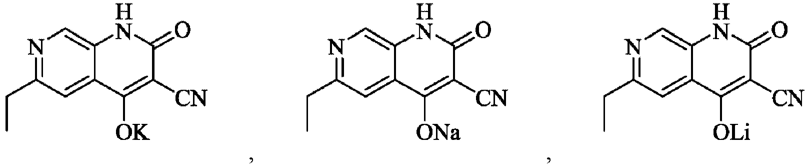 or
